# EUROPEAN PATENT SPECIFICATION

(11) **EP 3 041 458 B1**
(45) Date of publication and mention of the grant of the patent: **16.11.2016**
(21) Application number: 14752349.2
(22) Date of filing: 18.08.2014
(51) Int. Cl.: A61K 8/36, A61K 8/39, A61K 8/42, A61Q 15/00, A61K 8/92, A61K 8/02

(54) **ANTIPERSPIRANT COMPOSITIONS COMPRISING HYDROXYALKYL UREA**
SCHWEISSHEMMENDE ZUSAMMENSETZUNGEN
COMPOSITIONS ANTI-TRANSPIRANTES

(30) Priority: 02.09.2013 EP 13182561
(43) Date of publication of application: 13.07.2016
(73) Proprietor: Unilever PLC, London, Greater London EC4Y 0DY (GB); Unilever NV, 3013 AL Rotterdam (NL)
(72) Inventor: CIAMPI, Elisabetta, Leeds Yorkshire LS14 2AR (GB); MARRIOTT, Robert Edward, Wirral Merseyside CH63 3JW (GB); ROBERTS, Louise Jannette, Wirral Merseyside CH63 3JW (GB); TROW, Victoria Louise, Leeds Yorkshire LS14 2AR (GB); WHITEHEAD, Emma Jayne, Wirral Merseyside CH63 3JW (GB)
(74) Representative: McHugh, Paul Edward
(86) International application number: PCT/EP2014/067597
(87) International publication number: WO 2015/028341

(56) References cited:
- WO-A1-2008/145582
- GB-A- 2 431 580
- US-A1- 2012 276 033

## Description

### FIELD OF THE INVENTION

This invention relates to an antiperspirant composition. More particularly, the invention is directed to solid cosmetic antiperspirant compositions, which may take the form of a stick or soft solid.

### BACKGROUND OF THE INVENTION

Antiperspirant compositions are applied to the underarm area. It is desirable to include ingredients that provide a skin care benefit to this underarm area. The difficulty with formulating antiperspirant products to provide a skin care benefit is that many skin care benefit agents from the cosmetics field are difficult to formulate into antiperspirant products due to incompatibility with common ingredients in antiperspirants, such as the antiperspirant active, or have negative interactions such that sensory problems are introduced into the product.

One such class of ingredients is the hydroxyalkyl ureas.

WO 2008/145582 A1 discloses on page 15 an antiperspirant incorporating hydroxyethyl urea in combination with glycerol.

A problem with inclusion of this class of materials is that the resulting product feels gritty to the user, which is undesirable for a product that is applied to the skin.

GB 2 431 580 is concerned with the problem of grit formation in anhydrous antiperspirant compositions. It discloses non-gritty antiperspirant compositions comprising a low MW (up to 820) PEG. The active agent is added in the last step before filling the composition into a dispensing device.

We have found that a stable non-gritty solid antiperspirant product containing a hydroxyalkyl urea can be provided by inclusion of a carrier material selected from: triglyceride, polyalkylene glycol or a mixture thereof.

### SUMMARY OF THE INVENTION

In a first aspect, the invention is directed to a solid cosmetic antiperspirant composition comprising:-
(a) from 0.1 to 40 wt.% antiperspirant active;
(b) from 0.0005 to 3 wt.% hydroxyalkyl urea; and,
(c) from 0.1 to 10 wt.% of a carrier material selected from:
   i) a natural oil that comprises a triglyceride of an unsaturated carboxylic acid;
   ii) a polyalkylene glycol; or a mixture thereof.
   wherein the hydroxyalkyl urea is included as a premix with the carrier material

Preferably the solid composition is in the form of a solid antiperspirant stick. Preferably the natural oil comprises a triglyceride of an unsaturated carboxylic acid containing 1, 2, or 3 olefinic bonds.

Preferably the unsaturated carboxylic acid containing 1, 2, or 3 olefinic bonds that is part of the triglyceride contains from 14 to 22 carbon atoms. More preferably the triglyceride is a triglyceride of an unsaturated carboxylic acid containing 16-20 carbon atoms. Most preferably the triglyceride is a triglyceride of an unsaturated carboxylic acid containing 18 carbon atoms.

Preferably the natural oil comprises sunflower seed oil.

Preferably the polyalkylene glycol carrier material is selected from the group consisting of: polybutylene glycol, polypropylene glycol, polyethylene glycol and mixtures thereof.

Preferably the polyalkylene glycol carrier material has a molecular weight of up to 650 Daltons, more preferably from 360 to 460 Daltons.

Preferably the carrier material is selected from: sunflower seed oil, polyethylene glycol having a molecular weight of 360 to 460 Daltons and mixtures thereof.

Preferred hydroxyalkyl ureas have the following formula: 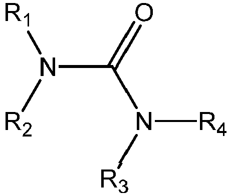 wherein R₁, R₂, R₃ and R₄ each independently represent: Hydrogen, a C₁₋₄ alkyl, or a C₂₋₆ hydroxyalkyl group, with the proviso that at least one of the groups R₁, R₂, R₃ and R₄ is a C₂₋₆ hydroxyalkyl group.

A particularly preferred hydroxyalkyl urea is a hydroxyethyl urea of structure:

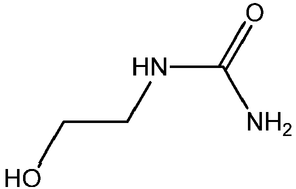

Preferably the antiperspirant stick composition comprises no more than 1 wt.% of water, more preferably the stick composition is anhydrous.

Preferably the hydroxyalkyl urea is included at a level of from the hydroxyalkyl urea is included at a level of from 0.0005 to 2.5 wt.%, more preferably from 0.001 to 2 wt.%.

A preferred antiperspirant active comprises an aluminium salt, preferably an aluminium/zirconium tetrachlorohydrex glycine complex.

A preferred solid antiperspirant composition according to the invention is in the form of an antiperspirant stick comprising:-
(a) from 5 to 40 wt.% antiperspirant active;
(b) from 0.0005 to 3 wt.% hydroxyalkyl urea;
(c) from 1 to 10 wt.% of a carrier material selected from:
   i) a natural oil that comprises a triglyceride of an unsaturated carboxylic acid;
   ii) a polyalkylene glycol; or a mixture thereof;
(d) from 5 to 35 wt.% of a non-volatile emollient;
(e) from 18 to 50 wt.% of a solvent oil; and,
(f) from 10 to 40 wt.% of a gelling agent
wherein the hydroxyalkyl urea is included as a premix with the carrier material

A preferred solid antiperspirant composition according to the invention is in the form of an antiperspirant soft solid composition comprising:-
(a) from 5 to 40 wt.% antiperspirant active;
(b) from 0.0005 to 3 wt.% hydroxyalkyl urea;
(c) from 0.1 to 10 wt.% of a carrier material selected from:
   i) a natural oil that comprises a triglyceride of an unsaturated carboxylic acid;
   ii) a polyalkylene glycol; or a mixture thereof;
(d) from 5 to 75 wt.% of a solvent; and,
(e) from 3 to 20 wt.% of a structurant.
wherein the hydroxyalkyl urea is included as a premix with the carrier material

The invention further relates in a second aspect to a method of production of a cosmetic antiperspirant stick composition comprising from 0.0005 to 3 wt% hydroxyalkyl urea, wherein the hydroxyalkyl urea is incorporated into the composition as a premix with a carrier material, selected from: a natural oil that comprises a triglyceride of an unsaturated carboxylic acid; polyalkylene glycol; or a mixture thereof.

### DETAILED DESCRIPTION OF THE INVENTION

As used herein, the term "comprising" means including, made up of, composed of, consisting and/or consisting essentially of.

All percentages quoted are wt.% based on total amount in the composition unless otherwise stated.

The invention is the finding that a stable non-gritty solid antiperspirant product containing a hydroxyalkyl urea can be provided by inclusion of a carrier material selected from: a natural oil that comprises a triglyceride of an unsaturated carboxylic acid; polyalkylene glycol; or a mixture thereof.

### Antiperspirant Active

The composition comprises from 0.1 to 40 wt.%, preferably from 5 to 40 wt.% of antiperspirant active. Antiperspirant active is preferably incorporated in an amount of at least 5 wt.%, more particularly from 20 to 30 wt.% of the composition. More preferably the antiperspirant active is present in an amount of from 22 to 27 wt.% of the composition.

Antiperspirant actives for use herein are often selected from astringent active salts, including, in particular, aluminium, zirconium and mixed aluminium/zirconium salts, including both inorganic salts, salts with organic anions and complexes. Preferred astringent salts include aluminium, zirconium and aluminium/zirconium halides and halohydrate salts, such as chlorohydrates and activated aluminium chlorohydrates.

Aluminium halohydrates are usually defined by the general formula Al₂(OH)ₓQ_{y}•wH₂O in which Q represents chlorine, bromine or iodine, x is variable from 2 to 5 and x+y=6 while wH₂O represents a variable amount of hydration. Zirconium actives can usually be represented by the empirical general formula: ZrO(OH)_{2n-nz}B_{z}•wH₂O in which z is a variable in the range of from 0.9 to 2.0 so that the value 2n-nz is zero or positive, n is the valency of B, and B is selected from the group consisting of chloride, other halide, sulfamate, sulfate and mixtures thereof. Possible hydration to a variable extent is represented by wH₂O. In one embodiment B represents chloride and the variable z lies in the range from 1.5 to 1.87. In practice, such zirconium salts are usually not employed by themselves, but as a component of a combined aluminium and zirconium-based antiperspirant.

The above aluminium and zirconium salts may have coordinated and/or bound water in various quantities and/or may be present as polymeric species, mixtures or complexes. In particular, zirconium hydroxy salts often represent a range of salts having various amounts of the hydroxy group. Aluminium zirconium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula CH₂(NH₂)COOH.

It is highly desirable to employ complexes of a combination of aluminium halohydrates and zirconium chlorohydrates together with amino acids such as glycine, examples of which are disclosed in U.S. Pat. No. 3,792,068 (Luedders et al).

Certain of those Al/Zr complexes are commonly called AZG in the literature. AZG actives generally contain aluminium, zirconium and chloride with an Al/Zr ratio in a range from 2 to 10, especially 2 to 6, an Al/Cl ratio from 2.1 to 0.9 and a variable amount of glycine. Actives of this type are available from suppliers that include Summit Reheis. In one preferred embodiment the active is enhanced activity or activated aluminium/zirconium halohydrate, in particular, activated aluminium-zirconium tetrachlorohydrex glycine (AAZG).

Other actives which may be utilized include astringent titanium salts, for example those described in GB 2299506A.

The proportion of solid particulate antiperspirant salt in a suspension composition normally includes the weight of any water of hydration and any complexing agent that may also be present in the solid active.

In one or more embodiments it is desirable that the mean particle size of the antiperspirant salts is within the range of 0.1 to 100 micron with a mean particle size that is often from 3 to 30 microns, more particularly from 5 to 35 microns, and certain embodiments of interest from 10 to 25 microns.

The particulate antiperspirant active may be present in the form of hollow spheres or dense particles (by which is meant particles which are not hollow) at the discretion of the manufacturer. To reduce the appearance of visible deposits on the skin to which the composition is applied or on clothing which comes into contact with the composition, it is preferable for the particles to be substantially free from hollows. Hollows can be eliminated by crushing the spheres.

The most preferred antiperspirant salt is an aluminium/zirconium tetrachlorohydrex glycine complex.

### Hydroxyalkyl Urea

Hydroxyalkyl ureas may be derived from urea (NH₂CONH₂).

Preferred hydroxyalkyl ureas have the formula: 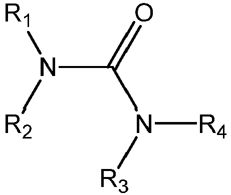 wherein R₁, R₂, R₃ and R₄ each independently represent: Hydrogen, a C₁₋₄ alkyl, or a C₂₋₆ hydroxyalkyl group, with the proviso that at least one of the groups R₁, R₂, R₃ and R₄ is a C₂₋₆ hydroxyalkyl group.

A particularly preferred hydroxyalkyl urea is hydroxyethyl urea, of structure:

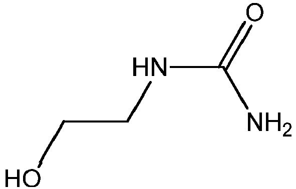

The hydroxyalkyl urea is present at a level of from 0.0005 to 3 wt.%, preferably from 0.0005 to 2.5 wt.%, more preferably from 0.001 to 2 wt.%, most preferably from 0.01 to 2 wt.%.

### Carrier Material

The composition comprises from 0.1 to 10 wt.% of a carrier material selected from: a natural oil that comprises a triglyceride of an unsaturated carboxylic acid; polyalkylene glycol; or a mixture thereof.

Preferred natural oils comprise a triglyceride of an unsaturated carboxylic acid containing 1, 2, or 3 olefinic bonds.

Preferably the unsaturated carboxylic acid containing 1, 2, or 3 olefinic bonds that is part of the triglyceride contains from 14 to 22 carbon atoms. More preferably the triglyceride is a triglyceride of an unsaturated carboxylic acid containing 16-20 carbon atoms. Most preferably the triglyceride is a triglyceride of an unsaturated carboxylic acid containing 18 carbon atoms.

The natural oil comprises a triglyceride.

The most preferred natural oil is sunflower seed *(Helianthus annuus*) oil.

Preferred polyalkylene glycol carriers comprise polyalkylene glycols selected from the group consisting of: selected from the group consisting of: polybutylene glycol, polypropylene glycol, polyethylene glycol and mixtures thereof.

The polyalkylene glycol preferably has a molecular weight of up to 650 Daltons, more preferably 500 Daltons, most preferably from 360 to 460 Daltons.

The preferred polyalkylene glycol is polyethylene glycol. The most preferred polyethylene glycol has from 4 to 12 ethylene oxide repeat units

The most preferred carrier is selected from: sunflower seed oil, polyethylene glycol having a molecular weight of 360 to 460 Daltons and mixtures thereof.

An especially preferred triglyceride comprises sunflower oil, ranging from those rich in oleic acid triglycerides to those rich in linoleic acid triglycerides, rich indicating that its content is higher than that of the other named acid.

A preferred ratio of hydroxyalkyl urea to carrier material is from 1:20 to 2:1, more preferably 1:10 to 1:1.

### Form of the Invention

The composition is a solid antiperspirant composition. These can preferably take the form of a solid stick, or a soft-solid.

Preferably the solid compositions comprise no more than 1 wt.% of water, more preferably the stick composition is anhydrous.

The stick composition is preferably solid, in that they retain their shape in the form of a stick without lateral support. The solid form is usually provided by way of a gellant.

The hardness of solid sticks is commonly measured using a conventional penetrometer test in which a Seta needle weighing 50g and having a tip angle of 9° 10' +/- 15' is allowed to drop for 5 seconds from surface contact with the test material. Desirably, the needle penetrates less than 30mm, preferably less than 20mm and especially up to 15mm, by virtue of the concentration of gellant or gellant mixture employed to solidify the composition.

An alternative method of measuring hardness employs a Stable Micro Systems TA.XT2i Texture Analyzer and Texture Expert Exceed™ software to generate the motion profile of a spherical probe employed in the method. A specific protocol involves a metal sphere, of diameter 9.5mm, is attached to the underside of a 5kg load cell, and positioned just above the sample surface. Under control of Expert Exceed™ software, the sphere is indented into the sample at an indentation speed of 0.05mm/s for a distance of 7 mm and reversed to withdraw the sphere from the sample at the same speed. Data comprising time(s) distance (mm) and force (N) is acquired at a rate of 25 Hz. The hardness H at a penetration of 4.76 mm is calculated using the formula:

H=F/A

in which H is expressed in N/mm², F is the load at the same travelled distance in N, and A is the projected area of the indentation in mm². This area can be calculated geometrically and is equal to the area of a diametral plane of the sphere, i.e., π x (4.76)²mm². A measured hardness of at least 0.5N/mm² indicates a solid stick.

Preferred solid antiperspirant stick compositions according to the invention comprise:-
(a) from 5 to 40 wt.% antiperspirant active;
(b) from 0.0005 to 3 wt.% hydroxyalkyl urea;
(c) from 0.1 to 10 wt.% of a carrier material selected from:
   i) a natural oil that comprises a triglyceride of an unsaturated carboxylic acid;
   ii) a polyalkylene glycol; or a mixture thereof;
(d) from 5 to 35 wt.% of a non-volatile emollient;
(e) from 18 to 50 wt.% of a solvent; and,
(f) from 10 to 40 wt.% of a gelling agent

Preferred gelling agents comprise: from 10 to 25 wt.% of a fatty alcohol wax; and, from 1 to 15 wt.% of hydrogenated castor oil.

Soft solids are widely used forms of antiperspirant products. The majority of commercially available soft solid products comprise no more than 1 wt.% of water, more preferably they are anhydrous suspensions that comprise antiperspirant active, carrier oil, and structurant. In such products, the antiperspirant active commonly comprises astringent aluminium salt, typically astringent aluminium/zirconium salt, suspended in a matrix formed by a combination of carrier oil and structurant.

Soft solids are structured compositions that are generally characterized as having a hardness of from 0.003 to 0.5 Newton/mm², and commonly from 0.003 or 0.01 up to 0.1 Newton/mm². It is measured in the same way as for the solid stick.

Preferred antiperspirant soft solid compositions according to the invention comprise:-
(a) from 5 to 40 wt.% antiperspirant active;
(b) from 0.0005 to 3 wt.% hydroxyalkyl urea;
(c) from 0.1 to 10 wt.% of a carrier material selected from:
   i) a natural oil that comprises a triglyceride of an unsaturated carboxylic acid;
   ii) a polyalkylene glycol; or a mixture thereof;
(d) from 5 to 75 wt.% of a solvent; and,
(e) from 3 to 20 wt.% of a structurant.
wherein the hydroxyalkyl urea is included as a premix with the carrier material.

In the stick or soft solid compositions, one or more further ingredients may be included, such as further oils, non-volatile emollient oil, silicone elastomers, gellants and fragrances.

Suitable solvents include silicone oils, which may be volatile or non-volatile. Particularly useful volatile oils are linear siloxanes containing from 3 to 9 silicon atoms, and cyclic siloxanes having from 4 to 6 silicon atoms, commonly referred to as cyclomethicone (e.g. cyclopentasiloxane, known as D5). Examples of commercially available volatile silicone oils include oils having grade designations 344, 345, 244, 245 and 246 from Dow Corning Corporation.

Particularly useful non-volatile silicone oils are polyalkyl siloxanes. Commercially available non-volatile silicone oils include products available under the trademarks Dow Corning 556 and Dow Corning 200 series

Solvents may be included at levels of from 5 to 75 wt.%, preferably 10 to 60 wt.%, more preferably 15 to 55 wt.%, or even 18 to 50 wt.%.

Suitable non-volatile emollient oils include the non-volatile silicone oils as previous described. Preferred non-volatile emollient oils are hydrocarbon oils, ester oils and ether oils.

Particularly preferred non-volatile emollient oils are C₈ to C₁₈ alkyl benzoate esters and PPG-14 butyl ether.

Non-volatile emollients, if present, may be present at a level of from 5 to 35 wt.%, preferably from 10 to 25 wt.%.

Silicone elastomers otherwise known as crosslinked dimethicone may be included, and are particularly useful in soft solid compositions. Elastomers tend to thicken oils, often by absorbing them and swelling. The elastomer is typically crosslinked by reacting a silicone hydride with an α-ω olefinically unsaturated dialkylene. If present, the elastomer is advantageously present at a concentration of at least 0.1 % up to 8%, and especially from 0.5% to 5% by weight of the antiperspirant composition. Elastomers are commercially available, for example from Dow Corning Inc and Shinetsu, and typically are supplied in a carrier oil that is frequently a cyclomethicone.

Preferred gellants include fatty alcohols having a melting range of from 55 to 75°C, and in many desirable embodiments, in the range of from 58 to 73°C. One or a blend of fatty alcohols can be employed, such as cetyl alcohol, stearyl alcohol, eicosyl alcohol and behenyl alcohol, or mixtures of any two or more thereof. Commercial fatty alcohols, though nominally and predominantly one specified alcohol often comprise a minor fraction, such as up to 5 or 6% by weight in total, of homologues differing by 2, 4 or even 6 carbons. When present, fatty alcohol is preferably present at a level of from 10 to 30 wt.%, preferably 11 to 20 wt.%, more preferably from 12.5 to 18.5 wt.%

Alternative gellants include waxes, preferably having a melting point in the range of from 70 to 95°C and especially from 75 to 90°C. Such waxes are often selected from hydrocarbon waxes and ester waxes, that can be derived from natural sources or synthesised. Suitable hydrocarbon waxes include mineral wax, microcrystalline wax, Montana wax, paraffin wax, and low molecular weight polyethylene, such as from 300 to 600 Daltons. Suitable ester waxes can be derived from unsaturated natural oils, such as plant-originating triglyceride oils by hydrogenation and optionally dehydroxylation (where the substituent contains at least one hydroxyl group as in castor oil). Suitable ester waxes include castor wax, candelilla wax, carnauba wax, beeswax and spermeceti wax. Natural waxes such as beeswax include a range of different chemical classes. Synthetic esters often comprise aliphatic monoesters containing at least 30 carbons, and indeed may be isolated natural products such as beeswax, or be derived from them or be the same compounds.

For solid sticks, a highly preferred gelling system is based on a fatty alcohol with a co-gellant wax as described above. Preferably the fatty alcohol would be present at a level of from 11 to 20 wt.%, and the wax would be present at a level of from 2 to 8 wt.%.

The most preferred gellant system for solid sticks is to use stearyl alcohol as the fatty alcohol in combination with hydrogenated castor oil as the wax co-gellant.

For soft solids, preferred structurants include wax structurants as hereinbefore described. The structurant is preferably present at a level of from 3 to 20 wt.%, more preferably 3 to 12 wt.%, and particularly 5 to 8 wt.%.

The composition can contain as perfume: free fragrance, a profragrance, encapsulated fragrance or fragrance that is associated with a host substrate such as cyclodextrin, or a mixture of any two or more of such perfume options.

Perfume (fragrance) if present, may be present at a level of from 0.25 to 2.5 wt.%.

### Dispensers

The compositions produced herein are suitable for dispensing from known cosmetic dispensers for solid antiperspirant formulations such as stick or soft solid dispensers. Such dispensers commonly comprise a barrel, often of round or oval transverse cross section, having an opening at a first end through which the composition is dispensed and an elevator at an opposed second end that can be advanced towards the first end. The elevator fits within the barrel. Commonly, the first end can be covered with a cap, conveniently dimensioned to push it over the exterior of the barrel.

For sticks, the opening is the full cross section of the barrel. The elevator can be advanced by insertion of finger within the barrel or by co-operation between a threaded spindle and aperture in the elevator, the spindle being rotated by either an externally protruding rotor wheel or by a pawl arrangement. Suitable dispensers for firm sticks are described, for example in US 4,232,977, US 4, 605,330, WO 09 818695, WO 09 603899, WO 09 405180, WO 09 325113, WO 09 305678, EP 1 040 445, US 5,997,202, US 5,897,263, US 5,496,122, US 5,275,496, US 6,598,767, US 6,299,369, or WO 2002/03830.

The compositions in the form of soft solid compositions, preferably the dispenser used has a plurality of openings through which the composition is dispensed.

### Examples

### Incorporation of Hydroxyethyl Urea (HEU) in a Solid Stick

The hydroxyethyl urea was sourced from Hydrovance as a 45-55% weight active in aqueous solution. Before use, it was freeze dried to yield a solid, which was then used in the formulations.

**Table 1 - Base Formulation**

| **Ingredient (wt.%) INCI Name** | **Trade Name** | **Supplier** | **Base Stick** |
|---|---|---|---|
| **Cyclomethicone** | PMX-0245 | Xiameter | Balance to 100% |
| **Aluminium Zirconium Tetrachlorohydrex Gly** | Reach 908 | Summit Reheis | 20.00 |
| **Stearyl Alcohol** | Lanette C18 Deo | Cognis | 17.00 |
| **C₁₂₋₁₅ Alkyl Benzoate** | Finsolv TN | Finetex / Innospec | 15.00 |
| **PPG-14 Butyl Ether** | Fluid AP | Dow Chemical | 9.00 |
| **Hydrogenated Castor Oil** | Castorwax MP80 | Vertellus Performance Materials | 4.00 |
| **Parfum** | | Givaudan | 1.20 |
| **Dimethicone** | DC 200 50cs PMX 200 50cs | Dow Corning Xiameter | 1.00 |
| **Polyethylene** | Performalene 400 | New Phase Technologies | 0.75 |
| **Silica** | Aerosil 200 | Evonik | 0.75 |
| **Steareth 100** | Brij S100 | Croda | 0.45 |
| **BHT** | Tenox BHT | Eastman | 0.05 |

To these base formulations, hydroxyethyl urea was added along with a carrier material (triglyceride, polyalkylene glycol or a mixture thereof).

The general method of preparation of the sticks was as follows:-
1. Oils (cyclomethicone, alkyl benzoate, PPG-14 butyl ether, dimethicone) and BHT were added together into an appropriate vessel, silica then added;
2. Contents of vessel were sheared until homogeneous (e.g. 7000rpm for 5 minutes);
3. Stearyl alcohol, wax, steareth 100 and polyethylene added to vessel;
4. Vessel heated to ca. 85°C to ensure complete dissolution of the waxes, and mixed (e.g. 250rpm using a Heidolph mixer) for 10 minutes;
5. HEU added along with carrier material (triglyceride, polyalkylene glycol or a mixture thereof) as a melt (heated to ca. 85°C)
6. Contents of vessel allowed to cool to 78°C, prior to slow addition of antiperspirant active. Contents of vessel mixed (e.g. 350rpm using a Heidolph mixer)
7. Temperature maintained above 70°C for at least 5 minutes following addition of all of the antiperspirant active
8. Fragrance added at 65°C
9. Formulation poured directly into stick barrel at ca. 62°C and allowed to set.

### Grittiness test

The resulting stick was tested for grit by a trained assessor. The assessment involved running an index finger over the domed surface of the stick formulation. The formulation was further assessed by removal of the domed surface of stick using a sharp knife, with the assessment involving running an index finger over the freshly cut stick surface of the stick formulation. These tests were repeated by running the stick over the back of the hand. The stick was then either assessed as gritty or non-gritty.

**Table 2 - Controls and Comparative examples**

| **Ingredient (wt.%) INCI Name** | **Control A** | **Control B** | **C** | **D** | **E** | **F** |
|---|---|---|---|---|---|---|
| **Cyclomethicone** (PMX-0245) | Balance to 100% | Balance to 100% | Balance to 100% | Balance to 100% | Balance to 100% | Balance to 100% |
| **Aluminium Zirconium Tetrachlorohydrex Gly** (Reach 908) | 20.00 | 20.00 | 20.00 | 20.00 | 20.00 | 20.00 |
| **Stearyl Alcohol** (Lanette C18 Deo) | 17.00 | 17.00 | 17.00 | 17.00 | 17.00 | 17.00 |
| **C₁₂₋₁₅ Alkyl Benzoate** (Finsolv TN) | 15.00 | 15.00 | 15.00 | 15.00 | 15.00 | 15.00 |
| **PPG-14 Butyl Ether** (Fluid AP) | 9.00 | 9.00 | 9.00 | 9.00 | 9.00 | 9.00 |
| **Hydrogenated Castor Oil** (Castorwax MP80) | 4.00 | 4.00 | 4.00 | 4.00 | 4.00 | 4.00 |
| **Parfum** | 1.20 | 1.20 | 1.20 | 1.20 | 1.20 | 1.20 |
| **Dimethicone** (DC 200 50cs) (PMX 200 50cs) | 1.00 | 1.00 | 1.00 | 1.00 | 1.00 | 1.00 |
| **Polyethylene** (Performalene 400) | 0.75 | 0.75 | 0.75 | 0.75 | 0.75 | 0.75 |
| **Silica** (Aerosil 200) | 0.75 | 0.75 | 0.75 | 0.75 | 0.75 | 0.75 |
| **Steareth 100** (Brij S100) | 0.45 | 0.45 | 0.45 | 0.45 | 0.45 | 0.45 |
| **BHT** (Tenox BHT) | 0.05 | 0.05 | 0.05 | 0.05 | 0.05 | 0.05 |
| **HEU** (Hydrovance) | | | 2 | 4 | 4 | 2 |
| **PEG-8** (Polyglykol 400) | | 2 | | 2 | | |
| **Sunflower Seed Oil** (Akosun) | | 0.5 | | | 2 | |
| **Glycerol** | | | | | | 2 |
| **Grittiness Test** | Non-Gritty | Non-Gritty | Gritty | Gritty | Gritty | Gritty |

These results show that incorporating hydroxyethyl urea (HEU) causes grit to occur. This poses a problem to incorporation of HEU. Formulation 'C' was made that included HEU without a carrier material. It resulted in a batch of sticks that did not include HEU in a homogeneous fashion. As the liquid formulation is poured into stick moulds, the early poured sticks were found to be HEU deficient, while the later sticks were HEU rich and were found to be very gritty. Attempts to solve this problem using a 'carrier material' vary depending upon the carrier chosen. For example, glycerol as a carrier still results in the unacceptable gritty stick (Formulation F). HEU incorporation at a level above 3 wt.%, even when the correct carrier material is chosen also results in an unacceptable gritty stick (Formulations D & E).

**Table 3 - Stick examples according to the Invention**

| **Ingredient (wt.%)** | **1** | **2** | **3** | **4** | **5** |
|---|---|---|---|---|---|
| **Cyclomethicone** (PMX-0245) | Balance to 100% | Balance to 100% | Balance to 100% | Balance to 100% | Balance to 100% |
| **Aluminium Zirconium Tetrachlorohydrex Gly** (Reach 908) | 20.00 | 20.00 | 20.00 | 20.00 | 20.00 |
| **Stearyl Alcohol** (Lanette C18 Deo) | 17.00 | 17.00 | 17.00 | 17.00 | 17.00 |
| **C₁₂₋₁₅ Alkyl** Benzoate (Finsolv TN) | 15.00 | 15.00 | 15.00 | 15.00 | 15.00 |
| **PPG-14 Butyl Ether** (Fluid AP) | 9.00 | 9.00 | 9.00 | 9.00 | 9.00 |
| **Hydrogenated Castor Oil** (Castorwax MP80) | 4.00 | 4.00 | 4.00 | 4.00 | 4.00 |
| **Parfum** | 1.20 | 1.20 | 1.20 | 1.20 | 1.20 |
| **Dimethicone** (DC 200 50cs) (PMX 200 50cs) | 1.00 | 1.00 | 1.00 | 1.00 | 1.00 |
| **Polyethylene** (Performalene 400) | 0.75 | 0.75 | 0.75 | 0.75 | 0.75 |
| **Silica** (Aerosil 200) | 0.75 | 0.75 | 0.75 | 0.75 | 0.75 |
| **Steareth 100** (Brij S100) | 0.45 | 0.45 | 0.45 | 0.45 | 0.45 |
| **BHT** (Tenox BHT) | 0.05 | 0.05 | 0.05 | 0.05 | 0.05 |
| **HEU** (Hydrovance) | 2 | 2 | 2 | 1 | 0.5 |
| **PEG-8** (Polyglykol 400) | | 2 | 2 | 2 | 2 |
| **Sunflower Seed Oil** (Akosun) | 2 | | 0.5 | | 0.5 |
| **Grittiness Test** | Non-Gritty | Non-Gritty | Non-Gritty | Non-Gritty | Non-Gritty |

As can be seen from the examples in tables 2 & 3, HEU is difficult to incorporate in a stick, but it can be incorporated at a level up to 3 wt.% by using a carrier material that is selected from: triglyceride, polyalkylene glycol or a mixture thereof.

**Table 4 - Soft solid examples according to the Invention**

| **Ingredient (wt.%) INCI Name** | **6** | **7** | **8** |
|---|---|---|---|
| **Cyclomethicone** | Balance to 100% | Balance to 100% | Balance to 100% |
| **Silicone Elastomer** | 4.00 | 4.00 | 4.00 |
| **Aluminium Zirconium Tetrachlorohydrex Gly** | 26.30 | 26.30 | 26.30 |
| **Fumed Silica** | 0.990 | 0.990 | 0.990 |
| **Dimethicone** | 8.00 | 10.00 | 10.00 |
| **Microcrystalline Wax** | 3.50 | 3.50 | 3.50 |
| **Paraffin Wax** | 3.50 | 3.50 | 3.50 |
| **Sunflower Seed Oil** | 0.1 | 1 | 0.5 |
| **HEU** | 0.001 | 1 | 0.001* |
| **Parfum** | 1.00 | 1.00 | 1.00 |

| | | | |
|---|---|---|---|
| * indicates that HEU was added as a 50% wt.% aqueous solution | | | |

## Claims

1. A solid cosmetic antiperspirant composition comprising:-
(a) from 0.1 to 40 wt.% antiperspirant active;
(b) from 0.0005 to 3 wt.% hydroxyalkyl urea; and,
(c) from 0.1 to 10 wt.% of a carrier material selected from:
i) a natural oil that comprises a triglyceride of an unsaturated carboxylic acid;
ii) a polyalkylene glycol; or a mixture thereof;
wherein the hydroxyalkyl urea is included as a premix with the carrier material.

2. A composition according to claim 1, wherein the solid antiperspirant composition is in the form of a solid antiperspirant stick.

3. A composition according to claim 1 or claim 2, wherein the natural oil comprises a triglyceride of an unsaturated carboxylic acid containing 1, 2, or 3 olefinic bonds.

4. A composition according to claim 3, wherein the natural oil comprises sunflower seed oil.

5. A composition according to claim 1, wherein the polyalkylene glycol carrier material is selected from the group consisting of: polybutylene glycol, polypropylene glycol, polyethylene glycol and mixtures thereof.

6. A composition according to claim 5, wherein the polyalkylene glycol carrier material has a molecular weight of up to 650 Daltons, preferably from 360 to 460 Daltons.

7. A composition according to claim 1, wherein the carrier material is selected from: sunflower seed oil; polyethylene glycol having a molecular weight of 360 to 460 Daltons; and mixtures thereof.

8. A composition according to any preceding claim, wherein the hydroxyalkyl urea has a formula: 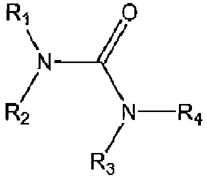 wherein R₁, R₂, R₃ and R₄ each independently represent: Hydrogen, a C₁₋₄ alkyl, or a C₂₋₆ hydroxyalkyl group, with the proviso that at least one of the groups R₁, R₂, R₃ and R₄ is a C₂₋₆ hydroxyalkyl group.

9. A composition according to claim 8, wherein the hydroxyalkyl urea is a hydroxyethyl urea of formula:

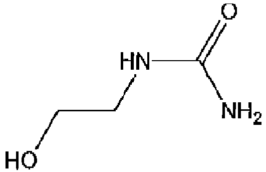

10. A composition according to any preceding claim, wherein the antiperspirant composition comprises no more than 1 wt.% of water, and preferably is anhydrous; and additionally comprises an aluminium salt, preferably an aluminium/zirconium tetrachlorohydrex glycine complex.

11. A composition according to any preceding claim, wherein the hydroxyalkyl urea is included at a level of from 0.0005 to 2.5 wt.%, more preferably from 0.001 to 2 wt.%.

12. A composition according to any preceding claim, wherein the composition is in the form of an antiperspirant stick composition comprising:-
(a) from 5 to 40 wt.% antiperspirant active;
(b) from 0.0005 to 3 wt.% hydroxyalkyl urea;
(c) from 0.1 to 10 wt.% of a carrier material selected from:
i) a natural oil that comprises a triglyceride of an unsaturated carboxylic acid;
ii) a polyalkylene glycol; or a mixture thereof.
(d) from 5 to 35 wt.% of a non-volatile emollient;
(e) from 18 to 50 wt.% of a solvent oil; and,
(f) from 10 to 40 wt.% of a gelling agent;
wherein the hydroxyalkyl urea is included as a premix with the carrier material.

13. A composition according to any one of claims 1 to 11, wherein the composition is in the form of an antiperspirant soft solid composition comprising:-
(a) from 5 to 40 wt.% antiperspirant active;
(b) from 0.0005 to 3 wt.% hydroxyalkyl urea;
(c) from 0.1 to 10 wt.% of a carrier material selected from:
i) a natural oil that comprises a triglyceride of an unsaturated carboxylic acid;
ii) a polyalkylene glycol; or a mixture thereof;
(d) from 5 to 75 wt.% of a solvent; and,
(e) from 3 to 20 wt.% of a structurant;
wherein the hydroxyalkyl urea is included as a premix with the carrier material.

14. A method of production of a solid cosmetic antiperspirant composition comprising from 0.0005 to 3 wt.% hydroxyalkyl urea, wherein the hydroxyalkyl urea is incorporated into the composition as a premix with a carrier material, selected from: a natural oil that comprises a triglyceride of an unsaturated carboxylic acid; polyalkylene glycol; or a mixture thereof.

## Patentansprüche

1. Feste kosmetische schweißhemmende Zusammensetzung, umfassend
(a) 0,1 bis 40 Gew.-% schweißhemmenden Wirkstoff,
(b) 0,0005 bis 3 Gew.-% Hydroxyalkylharnstoff und
(c) 0,1 bis 10 Gew.-% eines Trägermaterials, ausgewählt aus
i) einem natürlichen Öl, das ein Triglycerid einer ungesättigten Carbonsäure umfasst,
ii) einem Polyalkylenglykol
oder einer Mischung davon, wobei der Hydroxyalkylharnstoff als eine Vormischung mit dem Trägermaterial einbezogen ist.

2. Zusammensetzung nach Anspruch 1, wobei die feste schweißhemmende Zusammensetzung in Form eines festen schweißhemmenden Stifts vorliegt.

3. Zusammensetzung nach Anspruch 1 oder Anspruch 2, wobei das natürliche Öl ein Triglycerid einer ungesättigten Carbonsäure, die 1, 2 oder 3 olefinische Bindungen enthält, umfasst.

4. Zusammensetzung nach Anspruch 3, wobei das natürliche Öl Sonnenblumen(kern)öl umfasst.

5. Zusammensetzung nach Anspruch 1, wobei das Polyalkylenglykolträgermaterial aus der aus Polybutylenglykol, Polypropylenglykol, Polyethylenglykol und Mischungen davon bestehenden Gruppe ausgewählt ist.

6. Zusammensetzung nach Anspruch 5, wobei das Polyalkylenglykolträgermaterial ein Molekulargewicht bis zu 650 Dalton, vorzugsweise 360 bis 460 Dalton, aufweist.

7. Zusammensetzung nach Anspruch 1, wobei das Trägermaterial unter Sonnenblumen(kern)öl, Polyethylenglykol mit einem Molekulargewicht von 360 bis 460 Dalton und Mischungen davon ausgewählt ist.

8. Zusammensetzung nach irgendeinem vorhergehenden Anspruch, wobei der Hydroxyalkylharnstoff eine Formel 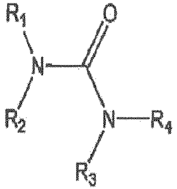 aufweist,
worin R₁, R₂, R₃ und R₄, jede unabhängig voneinander, darstellen: Wasserstoff, ein C₁₋₄-Alkyl oder eine C₂₋₆-Hydroxyalkylgruppe mit der Maßgabe, dass mindestens eine der Gruppen R₁, R₂, R₃ und R₄ eine C₂₋₆-Hydroxyalkylgruppe darstellt.

9. Zusammensetzung nach Anspruch 8, wobei der Hydroxyalkylharnstoff einen Hydroxyethylharnstoff der Formel 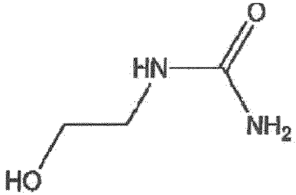 darstellt.

10. Zusammensetzung nach irgendeinem vorhergehenden Anspruch, wobei die schweißhemmende Zusammensetzung nicht mehr als 1 Gew.-% Wasser umfasst und vorzugsweise wasserfrei ist und zusätzlich ein Aluminiumsalz, vorzugsweise einen Aluminium/Zirconium-tetrachlorhydrexglycin-Komplex umfasst.

11. Zusammensetzung nach irgendeinem vorhergehenden Anspruch, wobei der Hydroxyalkylharnstoff in einer Konzentration von 0,0005 bis 2,5 Gew.-%, bevorzugter von 0,001 bis 2 Gew.%, einbezogen ist.

12. Zusammensetzung nach irgendeinem vorhergehenden Anspruch, wobei die Zusammensetzung in Form einer schweißhemmenden Stiftzusammensetzung vorliegt, umfassend:
(a) 5 bis 40 Gew.-% schweißhemmenden Wirkstoff,
(b) 0,0005 bis 3 Gew.-% Hydroxyalkylharnstoff,
(c) 0,1 bis 10 Gew.-% eines Trägermaterials, ausgewählt aus
i) einem natürlichen Öl, das ein Triglycerid einer ungesättigten Carbonsäure umfasst,
ii) einem Polyalkylenglykol
oder einer Mischung davon,
(d) 5 bis 35 Gew.-% eines nicht-flüchtigen Emollients,
(e) 18 bis 50 Gew.% eines Lösungsmittelöls und
(f) 10 bis 40 Gew.-% eines Geliermittels,
wobei der Hydroxyalkylharnstoff als eine Vormischung mit dem Trägermaterial einbezogen ist.

13. Zusammensetzung nach irgendeinem der Ansprüche 1 bis 11, wobei die Zusammensetzung in Form einer schweißhemmenden weichen, festen Zusammensetzung vorliegt, umfassend:
(a) 5 bis 40 Gew.-% schweißhemmenden Wirkstoff,
(b) 0,0005 bis 3 Gew.-% Hydroxyalkylharnstoff,
(c) 0,1 bis 10 Gew.-% eines Trägermaterials, ausgewählt aus
i) einem natürlichen Öl, das ein Triglycerid einer ungesättigten Carbonsäure umfasst,
ii) einem Polyalkylenglykol
oder einer Mischung davon,
(d) 5 bis 75 Gew.-% eines Lösungsmittels und
(e) 3 bis 20 Gew.-% eines Strukturierungsmittels,
wobei der Hydroxyalkylharnstoff als eine Vormischung mit dem Trägermaterial einbezogen ist.

14. Verfahren zur Herstellung einer festen kosmetischen schweißhemmenden Zusammensetzung, umfassend 0,0005 bis 3 Gew.-% Hydroxyalkylharnstoff, wobei der Hydroxyalkylharnstoff als eine Vormischung mit einem Trägermaterial, ausgewählt aus einem natürlichen Öl, das ein Triglycerid einer ungesättigten Carbonsäure, ein Polyalkylenglykol oder eine Mischung davon umfasst, einbezogen wird.

## Revendications

1. Composition d'antiperspirant cosmétique solide comprenant :
(a) de 0,1 à 40 % en masse d'actif d'antiperspirant ;
(b) de 0,0005 à 3 % en masse d'hydroxyalkylurée ; et
(c) de 0,1 à 10 % en masse d'un matériau de support choisi parmi :
i) une huile naturelle qui comprend un triglycéride d'un acide carboxylique insaturé ;
ii) un polyalkylène glycol ; ou un mélange de ceux-ci ;
dans laquelle l'hydroxyalkylurée est incluse comme un pré-mélange avec le matériau de support.

2. Composition selon la revendication 1, dans laquelle la composition d'antiperspirant solide est dans la forme d'un bâton d'antiperspirant solide.

3. Composition selon la revendication 1 ou la revendication 2, dans laquelle l'huile naturelle comprend un triglycéride d'un acide carboxylique insaturé contenant 1, 2 ou 3 liaisons oléfiniques.

4. Composition selon la revendication 3, dans laquelle l'huile naturelle comprend de l'huile de graine de tournesol.

5. Composition selon la revendication 1, dans laquelle le matériau de support de polyalkylène glycol est choisi dans le groupe constitué de : polybutylène glycol, polypropylène glycol, polyéthylène glycol et mélanges de ceux-ci.

6. Composition selon la revendication 5, dans laquelle le matériau de support de polyalkylène glycol présente une masse moléculaire jusqu'à 650 Daltons, de préférence de 360 à 460 Daltons.

7. Composition selon la revendication 1, dans laquelle le matériau de support est choisi parmi : une huile de graine de tournesol ; un polyéthylène glycol ayant une masse moléculaire de 360 à 460 Daltons ; et des mélanges de ceux-ci.

8. Composition selon l'une quelconque des revendications précédentes, dans laquelle l'hydroxyalkylurée présente une formule : 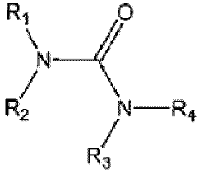
où R₁, R₂, R₃ et R₄ représentent chacun indépendamment :
l'hydrogène, un groupe alkyle en C₁₋₄, ou un groupe hydroxyalkyle en C₂₋₆, à condition qu'au moins un des groupes R₁, R₂, R₃ et R₄ soit un groupe hydroxyalkyle en C₂₋₆.

9. Composition selon la revendication 8, dans laquelle l'hydroxyalkylurée est une hydroxyéthylurée de formule :

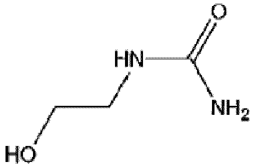

10. Composition selon l'une quelconque des revendications précédentes, dans laquelle la composition d'antiperspirant comprend au plus 1 % en masse d'eau, et est de préférence anhydre ; et comprend de plus un sel d'aluminium, de préférence un complexe d'aluminium/ zirconium tétrachlorohydrex glycine.

11. Composition selon l'une quelconque des revendications précédentes, dans laquelle l'hydroxyalkylurée est incluse à une teneur de 0,0005 à 2,5 % en masse, encore mieux de 0,001 à 2 % en masse.

12. Composition selon l'une quelconque des revendications précédentes, dans laquelle la composition est dans la forme d'une composition de bâton d'antiperspirant comprenant :
(a) de 5 à 40 % en masse d'actif d'antiperspirant ;
(b) de 0,0005 à 3 % en masse d'hydroxyalkylurée ;
(c) de 0,1 à 10 % en masse d'un matériau de support choisi parmi :
i) une huile naturelle qui comprend un triglycéride d'un acide carboxylique insaturé ;
ii) un polyalkylène glycol ; ou un mélange de ceux-ci
(d) de 5 à 35 % en masse d'un émollient non-volatil ;
(e) de 18 à 50 % en masse d'une huile de solvant ; et,
(f) de 10 à 40 % en masse d'un agent gélifiant ;
dans laquelle l'hydroxyalkylurée est incluse comme un pré-mélange avec le matériau de support.

13. Composition selon l'une quelconque des revendications 1 à 11, dans laquelle la composition est dans la forme d'une composition solide molle d'antiperspirant comprenant :
(a) de 5 à 40 % en masse d'actif d'antiperspirant ;
(b) de 0,0005 à 3 % en masse d'hydroxyalkylurée ;
(c) de 0,1 à 10 % en masse d'un matériau de support choisi parmi :
i) une huile naturelle qui comprend un triglycéride d'un acide carboxylique insaturé ;
ii) un polyalkylène glycol ; ou un mélange de ceux-ci ;
(d) de 5 à 75 % en masse d'un solvant ; et,
(e) de 3 à 20 % en masse d'un structurant ;
dans laquelle l'hydroxyalkylurée est incluse comme un pré-mélange avec le matériau de support.

14. Procédé de production d'une composition d'antiperspirant cosmétique solide comprenant de 0,0005 à 3 % en masse d'hydroxyalkylurée, dans lequel l'hydroxyalkylurée est incorporée dans la composition comme un pré-mélange avec un matériau de support, choisi parmi : une huile naturelle qui comprend un triglycéride d'un acide carboxylique insaturé ; un polyalkylène glycol ; ou un mélange de ceux-ci.
